# EUROPEAN PATENT APPLICATION

(11) **EP 4 663 766 A1**
(43) Date of publication of application: **17.12.2025**
(21) Application number: 24753438.1
(22) Date of filing: 09.02.2024
(51) Int. Cl.: C12P 7/6409, C12N 9/04, C12N 9/08, C12N 15/53, C12P 7/6418

(54) **METHOD FOR PRODUCING COMPOSITION CONTAINING PROCESSED FATTY ACID, AND USE FOR SAME**

(30) Priority: 10.02.2023 JP 2023018824
(71) Applicant: Amano Enzyme Inc., Nagoya-shi Aichi 460-8630 (JP)
(72) Inventor: KODAMA, Yusaku, Kakamigahara-shi, Gifu 509-0109 (JP)
(74) Representative: Witthoff Jaekel Steinecke Patentanwälte PartG mbB
(86) International application number: PCT/JP2024/004453
(87) International publication number: WO 2024/166995

(57) **Abstract**

It is an object of the present invention to provide a method for generating fatty acids having different carbon chain lengths by shortening the chain length of the fatty acids without using hydrogen peroxide. The present invention relates to a method for producing a processed fatty acid-containing composition, in which the method includes allowing fatty acid α-hydroxylase to act on a fatty acid-containing composition or a composition containing a compound having fatty acid as a partial structure in the presence of a substrate for oxidase and oxidase. In addition, the present invention also relates to a method for shortening the chain length of fatty acid, a method for producing a processed fatty acid-containing food product, and an enzyme agent for shortening the chain length of fatty acid.

## Description

### Technical Field

The present invention relates to a method for producing a processed fatty acid-containing composition and their applications. Specifically, the present invention relates to a method for producing a processed fatty acid-containing composition, a method for shortening the chain length of fatty acid, a method for producing a processed fatty acid-containing food product, and an enzyme agent for shortening the chain length of fatty acid.

### Background Art

Chemical synthesis of fatty acids is an extremely difficult technique, and most commercially available fatty acids are extracted from oil and fat raw materials. Moreover, most fatty acids in the bodies of mammals and birds have an even number of carbon atoms, and odd-numbered fatty acids having an odd number of carbon atoms exist in nature in very small numbers. The functions and commercial value of odd-numbered fatty acids have not been sufficiently recognized so far, and therefore, almost no methods for artificially synthesizing such odd-numbered fatty acids have been known.

In recent years, various techniques have been studied for the purpose of shortening the chain length of saturated fatty acids. For example, Non-Patent Document 1 discloses fatty acid α-hydroxylase (Cytochrome P450) derived from *Sphingomonas paucimobilis,* and it has been studied to shorten the chain length of saturated fatty acids by hydroxylating the α-position of fatty acids. Non-Patent Document 2 discloses a method for obtaining short-chain saturated fatty acids such as nonanoic acid by allowing Cytochrome P450 derived from Exiguobacterium sp. to act on myristic acid (saturated fatty acid). These documents disclose that hydrogen peroxide (H₂O₂) is added when the α-position of fatty acids is hydroxylated.

### Prior Art Documents

### Non-Patent Documents

Non-Patent Document 1: FEBS Letters 386 (1996) 252-254
Non-Patent Document 2: Catalysis Science & Technology, 2018, 8, 434-442

### Summary of Invention

### Objects to be Solved by the Invention

As described above, a method for shortening the chain length of saturated fatty acids has been studied, and a technique of adding hydrogen peroxide at that time has been disclosed. In this case, an excessive amount of hydrogen peroxide needs to be added when the α-position of fatty acids is hydroxylated to shorten the chain length of the fatty acids. However, hydrogen peroxide is harmful to human bodies, and thus, when fatty acids are used for food products, hydrogen peroxide needs to be removed. Accordingly, it has been desired to establish a technique of shortening the chain length of fatty acids without using hydrogen peroxide.

Thus, it is an object of the present invention to provide a method for generating fatty acids having different carbon chain lengths bu shortening the chain length of fatty acids without using hydrogen peroxide.

### Means for Solving the Objects

Examples of specific aspects of the present invention will be described below.

[1] A method for producing a processed fatty acid-containing composition, in which the method includes allowing fatty acid α-hydroxylase to act on a fatty acid-containing composition or a composition containing a compound having fatty acid as a partial structure in the presence of a substrate for oxidase and oxidase.
[2] The method for producing a processed fatty acid-containing composition according to [1], in which the fatty acid α-hydroxylase is Cytochrome P450.
[3] The method for producing a processed fatty acid-containing composition according to [1] or [2], in which the fatty acid α-hydroxylase consists of the amino acid sequence as set forth in SEQ ID No: 1 or an amino acid sequence equivalent thereto.
[4] The method for producing a processed fatty acid-containing composition according to any one of [1] to [3], in which the oxidase is carbohydrate oxidase.
[5] The method for producing a processed fatty acid-containing composition according to any one of [1] to [4], in which the substrate for the oxidase is glucose.
[6] The method for producing a processed fatty acid-containing composition according to any one of [1] to [5], in which
   the composition containing a compound having fatty acid as a partial structure is a glyceride-containing composition, and
   the method further includes allowing lipase to act on the glyceride-containing composition.
[7] A method for shortening the chain length of fatty acid, in which the method includes allowing fatty acid α-hydroxylase to act on fatty acid or a compound having fatty acid as a partial structure in the presence of a substrate for oxidase and oxidase.
[8] A method for producing a processed fatty acid-containing food product, in which the method includes allowing fatty acid α-hydroxylase to act on a fatty acid-containing food product or a food product containing a compound having fatty acid as a partial structure in the presence of a substrate for oxidase and oxidase.
[9] An enzyme agent for shortening the chain length of fatty acid, which contains fatty acid α-hydroxylase and oxidase.
[10] The enzyme agent for shortening the chain length of fatty acid according to [9], which further contains a substrate for the oxidase.
[11] The enzyme agent for shortening the chain length of fatty acid according to [9] or [10], in which the fatty acid α-hydroxylase is Cytochrome P450.
[12] The enzyme agent for shortening the chain length of fatty acid according to any one of [9] to [11], in which the fatty acid α-hydroxylase consists of the amino acid sequence as set forth in SEQ ID No: 1 or an amino acid sequence equivalent thereto.

[A] A processed fatty acid-containing composition, which is produced by the method for producing a processed fatty acid-containing composition according to any one of [1] to [6].
[B] The processed fatty acid-containing composition according to [A], which contains odd-numbered fatty acid.

### Advantageous Effects of Invention

According to the present invention, there can be provided a method for generating fatty acids having different carbon chain lengths by reducing the chain length of fatty acids without using hydrogen peroxide. In addition, according to the present invention, there are also provided a method for shortening the chain length of fatty acid and an enzyme agent for shortening the chain length of fatty acid.

### Embodiment of Carrying out the Invention

Hereafter, the present invention will be described in detail. The configuration requirements described below may be described based on representative embodiments or specific examples, but the present invention is not limited to such embodiments.

In the present description, the 20 types of amino acid residues in the amino acid sequences may be expressed by one-letter abbreviations in some cases. In that case, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, Aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P. Moreover, in the present description, in the amino acid sequences displayed, the N-terminus is at the left end and the C-terminus is at the right end.

In the present description, "non-polar amino acids" include alanine, valine, leucine, isoleucine, proline, methionine, phenylalanine and tryptophan. "Uncharged amino acids" include glycine, serine, threonine, cysteine, tyrosine, asparagine and glutamine. "Acidic amino acids" include aspartic acid and glutamic acid. "Basic amino acids" include lysine, arginine and histidine.

In the present description, "substitution" refers not only to the case where amino acid residue substitution is artificially introduced, but also to the case where amino acid residue substitution is introduced naturally, that is, the case where the amino acid residue was originally different. In the present description, the substitution of amino acid residues may be an artificial substitution, or a natural substitution, but an artificial substitution is preferable.

### (Method for producing processed fatty acid-containing composition)

The present embodiment relates to a method for producing a processed fatty acid-containing composition, in which the method includes allowing fatty acid α-hydroxylase to act on a fatty acid-containing composition or a composition containing a compound having fatty acid as a partial structure (hereinafter, in the present description, the fatty acid-containing composition and the composition containing a compound having fatty acid as a partial structure are collectively referred to as simply a "fatty acid-containing composition" at times) in the presence of a substrate for oxidase and oxidase. In the present embodiment, by allowing fatty acid α-hydroxylase to act on a fatty acid-containing composition in the presence of a substrate for oxidase and oxidase, the chain length of fatty acid is shortened, so that fatty acid having a shorter carbon chain length than the carbon chain length of the original fatty acid can be obtained.

Conventionally, when the α-position of fatty acid has been excessively hydroxylated to shorten the fatty acid, an excessive amount of hydrogen peroxide has needed to be added. However, hydrogen peroxide is harmful to human bodies, and thus, when fatty acids are used for food products, hydrogen peroxide needs to be removed. On the other hand, in the present embodiment, it has been succeeded to shorten fatty acid without using hydrogen peroxide. In addition, in the present embodiment, since a substrate for oxidase and oxidase are used, these are harmless to human bodies, and even in a case where fatty acids are used for food products, it is not necessary to remove oxidase and the like. Thus, in the present embodiment, a processed fatty acid-containing composition that is preferably used for food products can be produced.

Moreover, in the present embodiment, even in a case where a fatty acid-containing composition contains unsaturated fatty acid, the chain length of the fatty acid can be shortened. Conventionally, it has been believed that since unsaturated fatty acids have rigid double bonds in their structure, their access to enzymes is inhibited, compared to saturated fatty acids, which makes it difficult to shorten their chains. However, the present inventor has found that even in a case where the fatty acid-containing composition contains unsaturated fatty acid, the chain length of the fatty acid can be shortened. Furthermore, in the present embodiment, fatty acid having a short carbon chain length can be obtained, while the unsaturated binding portion is retained.

Further, in the present embodiment, odd-numbered fatty acids (C17:1, C15:1, C13:1, C11:1, C9:1, C7:1, C5:1, and C3:1), which are hardly present in nature and are difficult to be synthesized, can also be generated. In other words, according to the production method of the present embodiment, a processed fatty acid-containing composition that contains odd-numbered fatty acid can be obtained, and further, a processed fatty acid-containing composition that contains odd-numbered unsaturated fatty acid can also be obtained.

Besides, in the present description, when an unsaturated fatty acid is written as "Cx:y," it means that the unsaturated fatty acid has an x number of carbon atoms and a y number of carbon double bonds. For example, "C17:1" means that the unsaturated fatty acid has 17 carbon atoms and 1 carbon double bond.

### < Fatty acid-containing composition >

The fatty acid-containing composition contains fatty acid. The composition containing a compound having fatty acid as a partial structure contains a compound having fatty acid as a partial structure. Fatty acids are classified into long-chain fatty acids, medium-chain fatty acids, and short-chain fatty acids, based on their chain length. Long-chain fatty acids are fatty acids containing 11 or more carbon atoms, medium-chain fatty acids are fatty acids containing 7 to 10 carbon atoms, and short-chain fatty acids are fatty acids containing 6 or less carbon atoms. Moreover, fatty acids having an odd number of carbon atoms (3, 5, 7, 9, 11, 13, 15, 17, etc.) are referred to as odd-numbered fatty acids. Among them, the fatty acid-containing composition preferably contains long-chain fatty acid.

Fatty acids are classified into unsaturated fatty acids and saturated fatty acids. The fatty acid contained in the fatty acid-containing composition is not particularly limited, as long as fatty acid of interest is obtained. The fatty acids may be either saturated fatty acids or unsaturated fatty acids.

The unsaturated fatty acids are fatty acids having one or more carbon double bonds. Monovalent unsaturated fatty acids are unsaturated fatty acids having only one carbon double bond. On the other hand, polyvalent unsaturated fatty acids are unsaturated fatty acids having two or more carbon double bonds, and are divided into ω6 fatty acids and ω3 fatty acids. Specifically, the monovalent unsaturated fatty acids include myristoleic acid, palmitoleic acid, oleic acid, gondoic acid, erucic acid, nervonic acid, hexacosenoic acid, and octacosenoic acid. The ω3 polyvalent unsaturated fatty acids include α-linolenic acid, stearidonic acid, eicosatetraenoic acid, eicosapentaenoic acid, and docosahexaenoic acid. The ω6 polyvalent unsaturated fatty acids include linoleic acid, γ-linolenic acid, dihomo-γ-linolenic acid, arachidonic acid, and docosapentaenoic acid. In addition, the saturated fatty acids include lauric acid, myristic acid, palmitic acid, stearic acid, arachidic acid, behenic acid, and lignoceric acid. Other than the above-mentioned fatty acids, odd-numbered fatty acids can also be used as fatty acids. Besides, the fatty acid contained in the fatty acid-containing composition may be one type alone or may also be multiple types.

In the present embodiment, when the composition containing a compound having fatty acid as a partial structure is used as a substrate, the compound having fatty acid as a partial structure is preferably a fatty acid ester, and is more preferably glyceride. That is to say, the composition containing a compound having fatty acid as a partial structure is preferably a fatty acid ester-containing composition, and is more preferably a glyceride-containing composition. The glyceride-containing composition may be, for example, oil and fat. The glyceride-containing composition contains glyceride, and as such glyceride, triglyceride, diglyceride, or monoglyceride can be used. Besides, the glyceride is formed by binding at least one fatty acid to glycerin.

In the present embodiment, the fatty acid-containing composition may contain other optional components in addition to the fatty acid, or may consist of the fatty acid. Examples of such other optional components may include glycerin and salts (buffer salts). Moreover, the fatty acid-containing composition may also be a food product or a food material containing fatty acid or a compound having fatty acid as a partial structure. Examples of such food products or food materials may include edible oils (olive oil, safflower oil, sunflower oil, rapeseed oil, peanut oil, rice bran oil, etc.), edible meat (beef, chicken, pork, etc.), cheese, nuts (macadamia nuts, almonds, etc.), peanuts, sunflower seeds, eggs, milk, olives, and avocados.

Furthermore, the fatty acid-containing composition may contain, as other optional components, a pH adjuster, a preservative, a stabilizer, a thickener, an antioxidant, a surfactant, an emulsifier, a solubilizer, a dissolution aid, a bulking agent, a suspending agent, glycerin, salts (buffer salts), and the like. Examples of the pH adjuster may include an acetate buffer, a phosphate buffer, an MES buffer, a HEPES buffer, a PIPES buffer, a Bis-tris buffer, and a MOPS buffer.

The content of the fatty acid contained in the fatty acid-containing composition is preferably 0.001% by mass or more, more preferably 0.002% by mass or more, even more preferably 0.01% by mass or more, further preferably 0.1% by mass or more, even further preferably 1% by mass or more, still further preferably 5% by mass or more, and particularly preferably 10% by mass or more, based on the total mass of the fatty acid-containing composition. On the other hand, the content of the fatty acid may be 100% by mass, 90% by mass or less, 80% by mass or less, 70% by mass or less, 60% by mass or less, or 50% by mass or less, based on the total mass of the fatty acid-containing composition.

The form of the fatty acid-containing composition is not particularly limited as long as the enzyme reacts therewith, and it may be in any form, such as powders, a solid, gel, or a liquid (slurry). Of these, the fatty acid-containing composition is preferably in a liquid (slurry) form.

### < Fatty acid α-hydroxylase >

In the present embodiment, fatty acid α-hydroxylase is allowed to act on the fatty acid-containing composition in the presence of a substrate for oxidase and oxidase. The type and origin, etc. of the fatty acid α-hydroxylase are not particularly limited, as long as it is an enzyme exhibiting an action to hydroxylate the α-position of fatty acids. Examples of the fatty acid α-hydroxylase may include oxygenase and peroxygenase.

From the viewpoint of obtaining the effect of shortening fatty acids, the fatty acid α-hydroxylase is preferably oxygenase, and is more preferably cytochrome P450. The type of cytochrome P450 is not particularly limited, but examples thereof may include P450BSβ (CYP152A1) derived from *Bacillus subtilis,* P450Exα (CYP152N1) derived from Exiguobacterium sp., P450CLA (CYP152A2) derived from *Clostridium acetobutylicum,* and P450SPα (CYP152B1) derived from *Sphingomonas paucimobilis.* In the present embodiment, the fatty acid α-hydroxylase is preferably fatty acid α-hydroxylase in which α-position hydroxylation action is dominant compared to β-position oxidation action. Examples of such fatty acid α-hydroxylases may include P450Exα (CYP152N1) derived from Exiguobacterium sp. and P450SPα (CYP152B1) derived from *Sphingomonas paucimobilis.* Cytochrome P450 (P450Exα) derived from Exiguobacterium sp. is more preferable. These fatty acid α-hydroxylases may be used one type alone or in combination of multiple types.

The fatty acid α-hydroxylase is preferably an enzyme consisting of the amino acid sequence as set forth in SEQ ID No: 1 or an amino acid sequence equivalent thereto. Here, the term "equivalent amino acid sequence" refers to an amino acid sequence which is partially different from the reference amino acid sequence (the amino acid sequence as set forth in SEQ ID No: 1), but the difference does not substantially affect the functions of the protein (herein, fatty acid α-hydroxylation ability). Thus, an enzyme having an equivalent amino acid sequence catalyzes the fatty acid α-hydroxylation reaction. The degree of the activity is not particularly limited, as long as it can exhibit the function of the fatty acid α-hydroxylase. However, the activity is preferably at a degree equivalent to or higher than that of an enzyme consisting of the reference amino acid sequence. In addition, the "partial difference in an amino acid sequence" is produced, for example, as a result of a deletion or a substitution of one or more amino acids in the amino acids constituting the amino acid sequence, or an addition or an insertion of one or more amino acids into the amino acid sequence, or any given combination thereof. Such a partial difference in the amino acid sequence is acceptable, as long as the fatty acid α-hydroxylation activity is retained (in which the activity may be slightly fluctuated). As far as this condition is satisfies, the position of the amino acid sequence, in which the difference is found, is not particularly limited. In addition, such difference may be produced in multiple sites (places) on the amino acid sequence.

The number of amino acids providing such a partial difference in the amino acid sequence is, for example, a number corresponding to preferably less than about 30%, more preferably less than about 20%, even more preferably less than about 15%, further preferably less than about 10%, even further preferably less than about 7%, still further preferably less than about 5%, particularly preferably less than about 3%, further particularly preferably less than about 2%, and most preferably less than about 1%, with respect to the entire amino acids that constitute the amino acid sequence. Accordingly, the enzyme consisting of an equivalent amino acid sequence has an identity of, for example, about 70% or more, preferably about 80% or more, and more preferably about 85% or more, about 90% or more, about 93% or more, about 95% or more, about 97% or more, about 98% or more, and about 99% or more, to the reference amino acid sequence.

One typical example of the "partial difference in the amino acid sequence" is that a mutation (a change) is produced in the amino acid sequence, as a result of a deletion or a substitution of 1 to 40 (preferably 1 to 30, more preferably 1 to 10, even more preferably 1 to 7, further preferably 1 to 5, and still further preferably 1 to 3) amino acids in the amino acids constituting the amino acid sequence, or an addition or an insertion of 1 to 40 (preferably 1 to 30, more preferably 1 to 10, even more preferably1 to 7, further preferably 1 to 5, and still further preferably 1 to 3) amino acids into an amino acid sequence, or any given combination thereof.

For example, an equivalent amino acid sequence is obtained by the occurrence of conservative amino acid substitution in amino acid residues that are not essential for fatty acid α-hydroxylation ability. The term "conservative amino acid substitution" is used herein to mean that a certain amino acid residue is substituted with an amino acid residue having a side chain with similar properties. Amino acid residues are classified into several families, depending on the side chain thereof; namely, basic side chains (for example, lysine, arginine, and histidine), acidic side chains (for example, aspartic acid and glutamic acid), uncharged polar side chains (for example, glycine, asparagine, glutamine, serine, threonine, tyrosine, and cysteine), non-polar side chains (for example, alanine, valine, leucine, isoleucine, proline, phenylalanine, methionine, and tryptophan), β-branched side chains (for example, threonine, valine, and isoleucine), aromatic side chains (for example, tyrosine, phenylalanine, tryptophan, and histidine), etc. The conservative amino acid substitution is preferably a substitution occurring between amino acid residues in an identical family.

The identity (%) of two amino acid sequences can be determined, for example, by the following procedures. First, two sequences are aligned such that an optimal comparison can be made (for example, a gap may be introduced into a first sequence, so that the alignment with a second sequence may be optimized). When a molecule (amino acid residue) in a specific position of the first sequence is identical to a molecule in a corresponding position in the second sequence, it can be said that the molecules at the positions are identical to each other. The identity of the two sequences is a function of the number of identical positions common in the two sequences (i.e. identity (%) = the number of identical positions / total number of positions x 100), and preferably, the number and size of gaps used for optimization of the alignment are also taken into consideration.

Comparison of two sequences and determination of identity can be realized using mathematical algorithms. A specific example of the mathematical algorithm that can be utilized in comparison of sequences may be an algorithm that is described in Karlin and Altschul (1990) Proc. Natl. Acad. Sci. USA 87: 2264-68 and is modified in Karlin and Altschul (1993) Proc. Natl. Acad. Sci. USA 90: 5873-77, but the example of the mathematical algorithm is not limited thereto. The identity of amino acid sequences can be obtained, for example, by blastp (protein-protein BLAST) of National Center for Biotechnology Information (NCBI). As parameters, default parameters may be used. For example, using BLOSUM62 matrix, Gap Costs may be set to be

### Existence: 11 and Extension: 1.

The fatty acid α-hydroxylase may be a part of a larger protein (for example, a fusion protein). Examples of a sequence to be added to such a fusion protein may include sequences that are useful for purification, such as multiple histidine residues, and additional sequences that ensure stability during recombinant production.

The amount of the fatty acid α-hydroxylase used in the step of allowing the fatty acid α-hydroxylase to act on the fatty acid-containing composition is not particularly limited. The amount of the fatty acid α-hydroxylase per mg of fatty acid is, for example, preferably 0.01 U or more, and is more preferably 0.05 U or more, 0.1 U or more, 0.5 U or more, 1 U or more, 5 U or more, 10 U or more, 50 U or more, 100 U or more, 500 U or more, 1000 U or more, 5000 U or more, 10000 U or more, or 30000 U or more. On the other hand, the amount of the fatty acid α-hydroxylase used per mg of fatty acid is, for example, preferably 1000000 U or less, and is more preferably 100000 U or less, 60000 U or less, 40000 U or less, 20000 U or less, 10000 U or less, 5000 U or less, 2000 U or less, 1000 U or less, or 100 U or less.

The activity of the fatty acid α-hydroxylase can be determined from a change in the absorbance at a wavelength of 469 nm when a 5 mM 2,6-dimethoxyphenol solution (0.1 M potassium phosphate - 0.5 M potassium acetate buffer (pH 7.0), 0.5 mM hydrogen peroxide) is used as a substrate and is treated at 40°C. In the present description, the amount of the enzyme that oxidizes 1 nmol of the substrate in 1 minute is defined to be 1 unit (1 U).

### < Oxidase >

In the present embodiment, oxidase is used instead of hydrogen peroxide. The type, origin, etc. of the oxidase are not particularly limited, as long as it is an enzyme that generates hydrogen peroxide in association with a reaction that oxidizes a substrate. Examples of the oxidase may include carbohydrate oxidase, which uses a wide range of carbohydrates including glucose as substrates, glucose oxidase, which uses glucose as a substrate, alditol oxidase, which uses glycerin as a substrate, and oxalate oxidase, which uses oxalic acid as a substrate. Of these, the oxidase is preferably carbohydrate oxidase or glucose oxidase, and is more preferably carbohydrate oxidase. The type of carbohydrate oxidase is not particularly limited, but the carbohydrate oxidase may be, for example, carbohydrate oxidase derived from *Acremonium chrysogenum.*

In the present embodiment, a substrate for oxidase and oxidase are preferably used in combination. As mentioned above, examples of such a substrate for oxidase may include glucose, glycerin, oxalic acid. Among others, the substrate for oxidase is preferably glucose, and the oxidase is particularly preferably glucose oxidase having glucose as a substrate.

The amount of the oxidase used is not particularly limited. When carbohydrate oxidase is used, the amount of carbohydrate oxidase added per mg of fatty acid is, for example, preferably 0.01 U or more, 0.05 U or more, 0.1 U or more, 0.5 U or more, 1 U or more, 2 U or more, or 4 U or more. On the other hand, the amount of carbohydrate oxidase added per mg of fatty acid is, for example, preferably 5000 U or less, 1000 U or less, 500 U or less, 200 U or less, 100 U or less, 50 U or less, 40 U or less, 30 U or less, or 20 U or less.

When the oxidase is used, the amount of a substrate for the oxidase added is not particularly limited. The amount of a substrate for the oxidase added per mol of the fatty acid is, for example, preferably 0.1 mol or more, 0.5 mol or more, 1 mol or more, 2 mol or more, 5 mol or more, 7 mol or more, or 10 mol or more. On the other hand, the amount of a substrate for the oxidase added per mol of the fatty acid is, for example, preferably 1000 mol or less, 500 mol or less, 100 mol or less, 50 mol or less, or 30 mol or less.

When the glucose is used as a substrate for the carbohydrate oxidase, the amount of the glucose added per mol of fatty acid is, for example, preferably 0.1 mol or more, 0.5 mol or more, 1 mol or more, 5 mol or more, 10 mol or more, 15 mol or more, or 20 mol or more. On the other hand, the amount of glucose added per mol of fatty acid is, for example, preferably 1000 mol or less, 500 mol or less, 100 mol or less, 70 mol or less, 50 mol or less, or 30 mol or less.

Moreover, the amount of the glucose added per mg of the fatty acid is, for example, preferably 0.1 mg or more, 0.5 mg or more, 1 mg or more, 2 mg or more, 5 mg or more, 7 mg or more, or 10 mg or more. On the other hand, the amount of the glucose added per mg of the fatty acid is, for example, preferably 1000 mg or less, 500 mg or less, 100 mg or less, 50 mg or less, 30 mg or less, or 20 mg or less.

When carbohydrate oxidase or glucose oxidase is used as oxidase, the activity of the carbohydrate oxidase or the glucose oxidase can be measured by the following method. First, an appropriate amount of enzyme is weighed, a cooled potassium phosphate-sodium hydroxide buffer (0.1 mol/L) of pH 7.0 is added to the enzyme to dissolve or uniformly disperse it to prepare 50 mL of a solution, which is used as a sample solution. Then, 2.50 g of D(+)-glucose is weighed, and water is added to the glucose to dissolve it, so as to prepare 25 mL of a solution, which is used as a substrate solution. Then, 0.5 mL of the substrate solution, 2 mL of a potassium phosphate-sodium hydroxide buffer (0.1 mol/L, pH 7.0, containing phenol), 0.5 mL of a peroxidase reagent solution (25 units/mL), and 0.1 mL of a 4-aminoantipyrine solution (0.4% by mass) are placed in a quartz cell, and the resulting solution is warmed at 37°C for 10 minutes. Meanwhile, 0.1 mL of the sample solution is added to this solution, the obtained solution is fully mixed, and the obtained mixture is then warmed at 37°C to prepare a test solution. Instead of the sample solution, a potassium phosphate-sodium hydroxide buffer solution (0.1 mol/L) of pH 7.0 or water is used, and the same operations as those for preparation of the test solution are carried out to prepare a control solution. For the test solution and the control solution, the absorbance at a wavelength of 500 nm 2 minutes and 5 minutes after addition of the sample solution is measured. The amount of glucose oxidized is quantified from the molar extinction coefficient of the quinoneimine dye generated, and the amount of an enzyme required to oxidize 1 µmol of glucose per minute is defined to be 1 unit.

### < Optional components >

In the present embodiment, when a glyceride-containing composition is used as a substrate, lipase may be used in combination in the step of producing a processed fatty acid-containing composition. That is to say, the production method of the present embodiment may further include that the fatty acid-containing composition is a glyceride-containing composition, and that lipase is allowed to act on the glyceride-containing composition.

The type, origin, etc. of the lipase are not particularly limited, as long as the lipase is an enzyme that exhibits the action of hydrolyzing glyceride to release fatty acids. Examples of the lipase may include lipases derived from Rhizopus sp., Aspergillus sp., Mucor sp., Rhizomucor sp., Thermomyces sp., Pseudomonas sp., Geotrichums sp., Penicillium sp., and Candida sp., etc. The lipase derived from *Candida rugosa* is preferable.

The amount of the lipase used is not particularly limited. The amount of the lipase added per mg of glyceride is, for example, preferably 0.1 U or more, 0.5 U or more, 1 U or more, 5 U or more, 10 U or more, 20 U or more, 30 U or more, or 40 U or more. On the other hand, the amount of the lipase added per mg of glyceride is, for example, preferably 5000 U or less, 1000 U or less, 500 U or less, 200 U or less, 100 U or less, 90 U or less, 80 U or less, or 70 U or less.

### < Method for preparing fatty acid α-hydroxylase >

The method for preparing fatty acid α-hydroxylase may be, for example, a method of recovering fatty acid α-hydroxylase from the culture solution or the cell mass of, microorganisms, or recombinant microorganisms formed by introduction of the gene of the above-described enzyme therein. For example, when fatty acid α-hydroxylase-secreting microorganisms are used, after the cell mass has been recovered in advance from the culture medium by filtration, centrifugation, etc., as necessary, the enzyme can be separated and/or purified. On the other hand, when non-fatty acid α-hydroxylase-secreting microorganisms are used, after the cell mass has been recovered in advance from the culture medium, as necessary, the cell mass is disintegrated by a pressure treatment, an ultrasonic treatment, etc. to expose the enzyme, and the enzyme can be then separated and/or purified. As such a method of separating and/or purifying the enzyme, a known protein separation and/or purification method can be used without any particular limitations. Examples of the protein separation and/or purification method may include a centrifugation method, a UF concentration method, a salting-out method, and various chromatographic methods using ion exchange resins, etc. The purified fatty acid α-hydroxylase may be pulverized, as necessary, by freeze-drying, vacuum drying, spray drying, etc.

### < Conditions for production of processed fatty acid-containing composition >

In the present embodiment, fatty acid α-hydroxylase is allowed to act on a fatty acid-containing composition in the presence of a substrate for oxidase and oxidase. In this case, the substrate for oxidase acts with the oxidase, and the fatty acid α-hydroxylase acts with the fatty acid-containing composition. The step of allowing the oxidase to act on the substrate for oxidase and the step of allowing the fatty acid α-hydroxylase to act on the fatty acid may be different steps, or may also be steps that are simultaneously carried out in parallel.

In the step of allowing the oxidase to act on the substrate for oxidase and the step of allowing the fatty acid α-hydroxylase to act on the fatty acid-containing composition, the reaction time, the temperature, the pH of the reaction solution, etc. are not particularly limited. In addition, the reaction time, the temperature, the pH of the reaction solution, etc., in each step may be set to be different conditions. The reaction temperature in each step is, for example, 10°C to 70°C, preferably 20°C to 60°C, and more preferably 30°C to 50°C. The pH of the reaction solution is, for example, pH 4 to 10, preferably pH 5 to 9, and more preferably pH 6 to 8. The reaction time is, for example, 30 seconds to 24 hours, preferably 1 minute to 12 hours, and more preferably 2 minutes to 6 hours, 3 minutes to 2 hours, 4 minutes to 1 hour, or 5 minutes to 30 minutes. The shortening of the chain of the fatty acid can be more efficiently promoted under the above-described reaction conditions. These reaction conditions are appropriately selected, depending on the processed fatty acid-containing composition of interest. Besides, the optimal reaction conditions may be determined through preliminary experiments.

By using the production method of the present embodiment, a composition containing processed fatty acid having a shortened chain length (a shortened chain) can be produced. One aspect of the method for producing a processed fatty acid-containing composition of the present embodiment includes the following steps (1) and (2):
(1) a step of preparing a raw material containing fatty acid (a fatty acid-containing composition), and
(2) a step of treating the prepared raw material with fatty acid α-hydroxylase in the presence of a substrate for oxidase and oxidase.

It is to be noted that, after the step (2), (3) a step of inactivating the enzyme and/or (4) a step of recovering the fatty acid with a shortened chain length may be added.

Moreover, another aspect of the method for producing a processed fatty acid-containing composition of the present embodiment may include the following steps (1) to (3):
(1) a step of preparing a raw material containing fatty acid (a fatty acid-containing composition),
(2) a step of allowing oxidase to act on a substrate for oxidase, and
(3) a step of treating the raw material prepared in the above (1) with fatty acid α-hydroxylase in the presence of the acted product obtained in the above (2).

It is to be noted that, after the step (3), (4) a step of inactivating the enzyme and/or (5) a step of recovering the fatty acid with a shortened chain length may be added.

A further aspect of the production method of the present embodiment includes the following steps (1) to (3):
(1) a step of preparing a raw material containing glyceride to which fatty acid binds (a fatty acid-containing composition),
(2) a step of treating the prepared raw material with lipase, and
(3) a step of treating the lipase-treated raw material with fatty acid α-hydroxylase in the presence of a substrate of oxidase and oxidase.

It is to be noted that, after the step (3), (4) a step of inactivating the enzyme and/or (5) a step of recovering the saturated fatty acid with a shortened chain length may be added.

### (Processed fatty acid-containing composition)

The present embodiment may relate to a processed fatty acid-containing composition produced by the above-mentioned method for producing a processed fatty acid-containing composition. The processed fatty acid-containing composition contains processed fatty acid obtained by shortening the chain length of fatty acid (fatty acid with a shortened chain length). That is, the carbon chain length of the processed fatty acid is shortened, compared with that of the fatty acid. In addition, the processed fatty acid-containing composition preferably contains odd-numbered fatty acids, and such odd-numbered fatty acids may be odd-numbered unsaturated fatty acids (C17:1, C15:1, C13:1, C11:1, C9:1, C7:1, C5:1, and C3:1).

The processed fatty acid-containing composition may contain optional components that are contained in a fatty acid-containing composition. In addition, the form of the processed fatty acid-containing composition may be any form, such as powders, a solid, gel, or a liquid (slurry). Of these, the processed fatty acid-containing composition is preferably in a liquid (slurry) form.

In the present embodiment, the processed fatty acid-containing composition may be a processed fatty acid-containing food product. In addition, the fatty acid-containing composition may be a fatty acid-containing food product. The fatty acid-containing food product is a food product containing fatty acid or a compound having fatty acid as a partial structure. Examples of such food product may include processed oils and fats (olive oil, safflower oil, sunflower oil, rapeseed oil, peanut oil, rice bran oil, etc.), and fatty acid-containing food products (meat products, dairy products (cheese, processed milk, nut milk, and yogurt), egg products, etc.).

### (Enzyme agent)

The present embodiment relates to an enzyme agent for shortening the chain length of fatty acid, including fatty acid α-hydroxylase and oxidase. The enzyme agent for shortening the chain length of fatty acid contains the above-mentioned fatty acid α-hydroxylase and oxidase as active ingredients. Examples of the fatty acid α-hydroxylase and the oxidase may include the above-mentioned fatty acid α-hydroxylases and oxidases, and the preferred ranges are also the same.

The enzyme agent of the present embodiment preferably contains a substrate for oxidase, in addition to fatty acid α-hydroxylase and oxidase. The substrate for oxidase may be the same as the substrate for oxidase described above, and the preferred range is also the same.

The content of the fatty acid α-hydroxylase in the enzyme agent of the present embodiment is not particularly limited, and it can be appropriately set within a range in which catalytic activity for shortening the chain length of fatty acid is exhibited.

The enzyme agent of the present embodiment may contain other components in addition to the fatty acid α-hydroxylase and the oxidase to such an extent that they do not affect the effects of the present invention. Examples of such other components may include other enzymes other than the above-described fatty acid α-hydroxylase and oxidase, additives, culture residues generated in the step, etc. of preparing fatty acid α-hydroxylase, and the like. Moreover, the enzyme agent may be one agent or two or more agents.

Moreover, examples of other enzymes that may be contained in the enzyme agent of the present embodiment may include amylase (a-amylase, β-amylase, and glucoamylase), glucosidase (α-glucosidase and β-glucosidase), galactosidase (α-galactosidase and β-galactosidase), protease (acid protease, neutral protease, and alkaline protease), peptidase (leucine peptidase and aminopeptidase), lipase, esterase, cellulase, phosphatase (acid phosphatase and alkaline phosphatase), nuclease, deaminase, oxidase, dehydrogenase, glutaminase, pectinase, catalase, dextranase, transglutaminase, protein deamidase, pullulanase, peroxidase, and superoxide dismutase. These other enzymes may be one type or multiple types.

Examples of the additives that may be contained in the enzyme agent may include an excipient, a buffer agent, a suspending agent, a stabilizer, a preservative, an antiseptic, and a normal saline. Examples of the excipient may include starch, dextrin, maltose, trehalose, lactose, D-glucose, sorbitol, D-mannitol, white sugar, and glycerol. Examples of the buffer agent may include phosphate, citrate, and acetate. Examples of the stabilizer may include propylene glycol and ascorbic acid. Examples of the preservative may include phenol, benzalkonium chloride, benzyl alcohol, chlorobutanol, and methylparaben. Examples of the antiseptic may include ethanol, benzalkonium chloride, paraoxybenzoic acid, and chlorobutanol. These additives may be one type or multiple types.

The form of the enzyme agent of the present embodiment is not particularly limited, and examples thereof may include a powder form, a solid form, a gel form, a liquid form (slurry), and a form immobilized on a carrier.

### (Method for shortening chain length of fatty acid/method for producing processed fatty acid-containing food product)

The present embodiment may relate to a method for shortening the chain length of fatty acid, which includes allowing fatty acid α-hydroxylase to act on fatty acid or a compound having fatty acid as a partial structure in the presence of a substrate for oxidase and oxidase. By allowing the fatty acid α-hydroxylase to act on fatty acid in the presence of a substrate for oxidase and oxidase, fatty acid with a shortened chain length is obtained.

Moreover, the present embodiment may also relate to a method for producing a processed fatty acid-containing food product, which includes allowing fatty acid α-hydroxylase to act on a fatty acid-containing food product or a food product containing a compound having fatty acid as a partial structure in the presence of a substrate for oxidase and oxidase. The above-described food product is a food product or food material containing fatty acid or a compound having fatty acid as a partial structure, and examples of such food products or food materials may include edible oils (olive oil, safflower oil, sunflower oil, rapeseed oil, peanut oil, rice bran oil, etc.), edible meat (beef, chicken, pork, etc.), cheese, nuts (macadamia nuts, almonds, etc.), peanuts, sunflower seeds, eggs, milk, olives, and avocados.

### Examples

Hereinafter, the characteristics of the present invention will be more specifically described in the following examples. The materials, amounts used, proportions, treatment contents, treatment procedures, etc. shown in the following examples may be changed, as appropriate, without deviating from the gist of the present invention. Therefore, the scope of the present invention should not be interpreted limitedly by the specific examples shown below.

### (Materials used)

Enzymes: The enzymes used are as follows.
Fatty acid α-hydroxylase: Cytochrome P450 (P450Exα) derived from Exiguobacterium sp., AT1b
Carbohydrate oxidase: Carbohydrate oxidase derived from *Acremonium chrysogenum* (manufactured by Amano Enzyme Inc.)

**[Table 1]**

| **Unsaturated fatty acid** | **Abbreviation** | **Examples of food products containing unsaturated fatty acid** | **Manufacturer** | **Content** |
|---|---|---|---|---|
| Oleic acid | C18:1 | Sunflower oil, olive oil, rapeseed oil | Tokyo Chemical Industry Co., Ltd. | > 99.0% |

| **Saturated fatty acids** | **Abbreviation** | **Examples of food products containing large amounts of saturated fatty acids** | **Manufacturer** | **Content** |
|---|---|---|---|---|
| Stearic acid | C18 | Beef, pork, chocolate | Tokyo Chemical Industry Co., Ltd. | > 98% |
| Myristic acid | C14 | Palm oil, coconut, butter | Wako | 98% |

### (Method of obtaining enzyme)

The gene for fatty acid peroxygenase (P450Exα gene: SEQ ID No: 2) derived from the synthesized Exiguobacterum sp. AT1b was amplified by PCR. The amplified DNA was inserted into an *E. coli* expression vector (pET28a), and the resulting vector was then introduced into an *E. coli* BL21 strain by chemical transformation. The obtained transformant was inoculated into a liquid medium (pH 7.0) containing yeast extract, peptone and glycerol, and was then cultured at 25°C for 24 hours with aeration and agitation. After recovering a cell mass from the culture solution, the cell mass was disrupted with a bead shocker, and the enzyme was then extracted. The obtained extract was concentrated by ultrafiltration and was purified with an affinity column to obtain an enzyme solution.

### (Method of measuring enzyme concentration)

The concentration of the enzyme (P450Exα) in the enzyme solution was measured by the pyridine ferrohemochrome method. First, pyridine was added into a sodium hydroxide solution to a final concentration of 20 mM, and an arbitrary amount of the enzyme solution was then added and mixed. Thereby, the heme b in P450Exα was allowed to release. Thereafter, dithionite was added thereto to reduce the heme b. The pyridine was coordinated with the heme b in the reduced state, so that absorption was observed at a characteristic wavelength of 557 nm. The P450Exα concentration in the enzyme solution was calculated from the molar extinction coefficient of 34.7 mM⁻¹ cm⁻¹.

### (Method of measuring enzyme activity of P450Exα)

0.1 M Potassium phosphate, 0.5 M potassium acetate, and 0.5 mM hydrogen peroxide were placed in a plastic cell, and the obtained mixture was then incubated at 40°C for 30 minutes. Thereafter, 5 mM 2,6-dimethoxyphenol was added, and the obtained mixture was then incubated at 40°C for 1 minute. Subsequently, an enzyme solution diluted to an appropriate concentration was added to the reaction mixture, and a change in the absorbance at a wavelength of 469 nm was measured for 3 minutes using a spectrophotometer. The reaction rate was obtained by calculating a difference in the absorbance between 130 and 150 seconds, and the amount of enzyme that oxidizes 1 nmol of substrate per minute was calculated as 1 unit (1 U).

### (Reference Examples 1 to 3)

### (1) Fatty acid-shortening reaction by combined use of P450Exα and oxidase

### (Method)

A potassium phosphate buffer (pH 7) (concentration during reaction: 0.1 M) and each type of fatty acid used as a substrate (myristic acid, stearic acid, or oleic acid) (concentration during reaction: 500 µM) were added to a 6 cc vial to a liquid volume during the reaction of 0.5 mL, and the obtained mixture was then left at rest at 40°C for 5 minutes. To the reaction mixture, P450Exα (concentration during reaction: 1080 U/mL) was added, and the thus obtained mixture was then incubated at 40°C for 1 minute. Thereafter, hydrogen peroxide was added to a concentration during the reaction of 10 mM, and the obtained mixture was then reacted at 40°C for 1 hour. It is to be noted that the substrate was dissolved in ethanol, and that the final concentration of ethanol in the reaction solution was set to be 1%. After each reaction time had passed, 1 mL of chloroform and 5 µL of 6N HCl were added to 0.5 mL of the reaction solution, and the obtained mixture was thoroughly stirred. After that, 300 µL of the chloroform layer was fractionated into a new vial, 300 µL of BSTFA-TMCS (99 : 1) (Tokyo Chemical Industry Co., Ltd.) was then added to the vial, and the obtained mixture was then left at rest for 1 hour or more. Thereafter, 1 µL of this solution was injected into a GC-MS, and the abundance percentage of fatty acids after the reaction was determined. It is to be noted that the abundance percentage was calculated from the percentage of individual fatty acid peaks to the sum of the peaks corresponding to m/z = 119 (fatty acid fragments) in the GC-MS.

### < Conditions for GC-MS analysis >

| | |
|---|---|
| Column | DB-5 |
| Carrier gas | He |
| Column flow rate | 2.14 mL |
| Purge flow rate | 6.0 mL |
| Split ratio | 16.5 |
| Interface temperature | 200°C |
| Vaporizer temperature | 250°C |
| Ion source temperature | 200°C |
| Column temperature | 50°C (1 min)-10°C/min (15 min)-50°C/min (2 min)-300°C (12 min) |

### (Examples 1 to 6)

A potassium phosphate buffer (pH 7) (concentration during reaction: 0.1 M), glucose (concentration during reaction: 0.2% by mass), and each type of fatty acid used as a substrate (myristic acid, stearic acid, or oleic acid) (concentration during reaction: 500 µM) were added to a 6 cc vial to a liquid volume during the reaction of 0.5 mL, and the obtained mixture was then left at rest at 40°C for 5 minutes. To the reaction mixture, P450Exα (concentration during reaction: 1080 U/mL) and carbohydrate oxidase (concentration during reaction: 1.5 U/mL or 0.75 U/mL) were added, and the obtained mixture was then reacted at 40°C for 1 hour. It is to be noted that the substrate was dissolved in ethanol, and that the final concentration of ethanol in the reaction solution was set to be 1%. After each reaction time had passed, 1 mL of chloroform and 5 µL of 6N HCl were added to 0.5 mL of the reaction solution, and the obtained mixture was thoroughly stirred. After that, 300 µL of the chloroform layer was fractionated into a new vial, 300 µL of BSTFA-TMCS (99 : 1) (Tokyo Chemical Industry Co., Ltd.) was then added to the vial, and the obtained mixture was then left at rest for 1 hour or more. Thereafter, 1 µL of this solution was injected into a GC-MS, and the same analysis as that described above was carried out, so that the abundance percentage of fatty acids was calculated.

Table 1 shows the percentage of fatty acids after treatment, when myristic acid was used as a substrate; Table 2 shows the percentage of fatty acids after treatment, when stearic acid was used as a substrate; and Table 3 shows the percentage of fatty acids after treatment, when oleic acid was used as a substrate.

**[Table 2]**

| | Ref. Ex. 1 | Example 1 | Example 2 |
|---|---|---|---|
| P450Exα (U/mL) | 1080 | 1080 | 1080 |
| Hydrogen peroxide (mM) | 10 | - | - |
| Carbohydrate oxidase (U/mL) | - | 0.75 | 1.5 |
| Glucose (%) | - | 0.2 | 0.2 |
| C7 | 0 | 0 | 0 |
| C8 | 1 | 3 | 1 |
| C9 | 5 | 1 | 2 |
| C10 | 30 | 9 | 15 |
| C11 | 47 | 38 | 44 |
| C12 | 12 | 33 | 26 |
| C13 | 2 | 9 | 6 |
| C14 | 3 | 7 | 5 |

**[Table 3]**

| | Ref. Ex. 2 | Example 3 | Example 4 |
|---|---|---|---|
| P450Exα (U/mL) | 1080 | 1080 | 1080 |
| Hydrogen peroxide (mM) | 10 | - | - |
| Carbohydrate oxidase (U/mL) | - | 0.75 | 1.5 |
| Glucose (%) | - | 0.2 | 0.2 |
| C7 | 1 | 1 | 0 |
| C8 | 1 | 0 | 1 |
| C9 | 1 | 1 | 1 |
| C10 | 3 | 1 | 1 |
| C11 | 7 | 3 | 3 |
| C12 | 10 | 10 | 8 |
| C13 | 7 | 6 | 5 |
| C14 | 5 | 6 | 5 |
| C15 | 3 | 2 | 2 |
| C16 | 31 | 32 | 39 |
| C17 | 3 | 4 | 3 |
| C18 | 34 | 37 | 36 |

**[Table 4]**

| | Ref. Ex. 3 | Example 5 | Example 6 |
|---|---|---|---|
| P450Exα (U/mL) | 1080 | 1080 | 1080 |
| Hydrogen peroxide (mM) | 10 | - | - |
| Carbohydrate oxidase (U/mL) | - | 0.75 | 1.5 |
| Glucose (%) | - | 0.2 | 0.2 |
| C13:1 | 11 | 12 | 13 |
| C14:1 | 37 | 37 | 33 |
| C15:1 | 23 | 28 | 24 |
| C16:1 | 12 | 16 | 13 |
| C17:1 | 0 | 0 | 0 |
| C18:1 | 17 | 7 | 17 |

### (Results)

From the above-described results, it was clarified that equivalent effects can be obtained by adding oxidase and a substrate thereof, instead of hydrogen peroxide. In addition, according to the present invention, it was found that odd-numbered fatty acids, which are hardly present in nature and are difficult to be synthesized, can be generated.

### Industrial Applicability

According to the present invention, equivalent effects can be obtained by adding oxidase and a substrate thereof, instead of hydrogen peroxide. In addition, the present invention provides a technique of generating odd-numbered fatty acids, which are hardly present in nature and are difficult to be synthesized, and this is a technique that is useful, in particular, in the field of pharmaceutical products and in the field of food products.
SEQ ID No: 1: The amino acid sequence of Exiguobacterium sp. AT1b-derived Cytochrome P450 (P450Exα)
SEQ ID No: 2: The nucleotide sequence of Exiguobacterium sp. AT1b-derived Cytochrome P450 (P450Exα) gene

SEQ ID No: 1
MGKVIPKQEGLDHSVDFLREGYLFVANRRKSFQSNIFESRLLGERVICLGGEEAA
EVFYDANKFTRQDAAPKRLLKTLFGEGGVQTLDGSEHTHRKQMFMSLMTKEN
IDRLLRLTYREWNQIERMGEEIVLYDIAQEVLMKAVCEWSGVPLAKEEVGKRTE
EMRLLFESGTSLGPTYLQGRKARSSAEVWIRQMVKEVRSNRLLPNEHTALYEFS
WHRDESGELLPEEVVAVEVLNILRPTVAISVYVLFTVLALHQFPDVKEQVERGE
VSKTEFVQEVRRFYPFFPVAAARVKTDFEWDGYAFPEGTLTLLDLYGTNHDVSI
WTEPDRFDPSRFKDWKESPFNFIPQGGGDVDFGHRCAGEHVTIAILAQVIELFTK
EYAYTVPPQDLSYSFVDMPSLPKSKLRLTHLTRNQ

SEQ ID No: 2
atgggtaaagttatcccgaaacaggaaggcctggatcactctgtggactttctgcgcgagggctatctgtttgtggcaaaccgc
cgtaaatctttccagtctaacatcttcgagagccgcctgctgggcgagcgtgttatctgcctgggtggcgaagaagctgcagag
gtgttttatgacgcgaacaaattcacccgtcaggacgcggcaccgaaacgtctgctgaaaaccctgttcggtgaaggcggtgt
gcagaccctggacggttccgaacacacccaccgcaagcagatgttcatgagcctgatgaccaaagaaaacattgatcgcctg
ctgcgcctgacttatcgtgaatggaaccagatcgaacgtatgggtgaggaaatcgttctgtacgacatcgcacaggaggtgct
gatgaaagcagtgtgcgaatggtctggtgttccgctggctaaggaagaagtgggtaagcgtaccgaagaaatgcgtctgctgt
tcgaatctggtacctctctgggtccgacctatctgcagggtcgcaaagcgcgttcttctgcggaagtttggattcgtcagatggtt
aaagaagttcgctctaaccgtctgctgccgaacgaacatactgcgctgtatgaattctcttggcaccgtgatgagagcggtgaa
ctgctgccggaagaggtggtggctgttgaagtgctgaacattctgcgtccgactgttgctatctctgtgtacgtgctgtttaccgt
gctggcgctgcaccagtttccggacgttaaggaacaggttgagcgtggtgaggtttctaaaactgagtttgtgcaggaagtgc
gccgtttctacccgtttttcccggttgcagcagctcgtgttaaaaccgatttcgaatgggatggctatgcgttcccggaaggtacc
ctgactctgctggacctgtacggtactaaccacgacgtgtctatttggaccgaaccggatcgtttcgacccgtctcgtttcaaag
attggaaagaatctccgttcaacttcatcccgcagggcggtggtgatgttgattttggccaccgttgcgcgggtgagcacgttac
catcgcaatcctggcgcaggttatcgaactgttcaccaaggagtatgcgtataccgttccgccgcaggatctgtcttactctttcg
ttgacatgccgtccctgccgaaaagcaaactgcgtctgactcacctgacccgtaaccagtaa

## Claims

1. A method for producing a processed fatty acid-containing composition, in which the method includes allowing fatty acid α-hydroxylase to act on a fatty acid-containing composition or a composition containing a compound having fatty acid as a partial structure in the presence of a substrate for oxidase and oxidase.

2. The method for producing a processed fatty acid-containing composition according to claim 1, in which the fatty acid α-hydroxylase is Cytochrome P450.

3. The method for producing a processed fatty acid-containing composition according to claim 1, in which the fatty acid α-hydroxylase consists of the amino acid sequence as set forth in SEQ ID No: 1 or an amino acid sequence equivalent thereto.

4. The method for producing a processed fatty acid-containing composition according to claim 1, in which the oxidase is carbohydrate oxidase.

5. The method for producing a processed fatty acid-containing composition according to claim 1, in which the substrate for the oxidase is glucose.

6. The method for producing a processed fatty acid-containing composition according to claim 1, in which
the composition containing a compound having fatty acid as a partial structure is a glyceride-containing composition, and
the method further includes allowing lipase to act on the glyceride-containing composition.

7. A method for shortening the chain length of fatty acid, in which the method includes allowing fatty acid α-hydroxylase to act on fatty acid or a compound having fatty acid as a partial structure in the presence of a substrate for oxidase and oxidase.

8. A method for producing a processed fatty acid-containing food product, in which the method includes allowing fatty acid α-hydroxylase to act on a fatty acid-containing food product or a food product containing a compound having fatty acid as a partial structure in the presence of a substrate for oxidase and oxidase.

9. An enzyme agent for shortening the chain length of fatty acid, which contains fatty acid α-hydroxylase and oxidase.

10. The enzyme agent for shortening the chain length of fatty acid according to claim 9, which further contains a substrate for the oxidase.

11. The enzyme agent for shortening the chain length of fatty acid according to claim 9, in which the fatty acid α-hydroxylase is Cytochrome P450.

12. The enzyme agent for shortening the chain length of fatty acid according to claim 9, in which the fatty acid α-hydroxylase consists of the amino acid sequence as set forth in SEQ ID No: 1 or an amino acid sequence equivalent thereto.
